# EUROPEAN PATENT SPECIFICATION

(11) **EP 3 302 080 B1**
(45) Date of publication and mention of the grant of the patent: **09.01.2019**
(21) Application number: 16729209.3
(22) Date of filing: 27.05.2016
(51) Int. Cl.: A23C 21/00, A23C 1/00, C07H 3/04, C13K 5/00

(54) **SECOND PASS LACTOSE CRYSTALLIZATION**
LACTOSEKRISTALLISATION MIT ZWEITEM DURCHGANG
CRISTALLISATION DE LACTOSE DE SECOND PASSAGE

(30) Priority: 29.05.2015 DK 201500313; 07.08.2015 DK 201500449
(43) Date of publication of application: 11.04.2018
(73) Proprietor: Spx Flow Technology Danmark A/S, 2860 Søborg (DK)
(72) Inventor: ANDERSEN, Gert, 3550 Slangerup (DK); SCHØNFELDT, Henrik, 2820 Gentofte (DK); WAGNER, Peter, 2970 Hørsholm (DK)
(74) Representative: Nordic Patent Service A/S
(86) International application number: PCT/EP2016/061976
(87) International publication number: WO 2016/193138

(56) References cited:
- WO-A1-02/50089
- WO-A1-2014/141164
- US-A- 2 778 750
- US-A- 4 404 038
- GUU Y K: "Nanofiltration Concentration Effect on the Efficacy of Lactose Crystallisation", JOURNAL OF FOOD SCIENCE, WILEY-BLACKWELL PUBLISHING, INC, US, vol. 57, no. 3, 1 May 1992 (1992-05-01), pages 735-739, XP002126233, ISSN: 0022-1147

## Description

### FIELD OF THE INVENTION

The application generally relates to a method of obtaining solid lactose from one or more aqueous solutions comprising lactose and an arrangement configured for executing the method.

More specifically the invention relates to a method of obtaining solid lactose derived from whey or whey permeate.

The invention is described with the help of a method for obtaining a solid crystallized lactose product.

### BACKGROUND OF THE INVENTION

Crystallization is an important processing step in the manufacture of alpha lactose monohydrate from liquid solutions, in particular whey solutions. The efficient recovery of alpha lactose monohydrate is an important issue for industry and in particular, for the dairy industry. During crystallization lactose is typically lost in one or more waste streams, reducing the lactose yield. Mother liquor typically comprises about 17% to 21% lactose by weight of total solids, which often is not recovered but goes to waste instead.

In the art, it is known to recycle some of the lactose waste streams continuously. For example, the wash water from sieving is sometimes recycled, since it is very pure.

WO2014141164 discloses a method of obtaining lactose from a lactose-containing liquid source comprising providing a lactose-containing liquid source, crystallizing the lactose in the lactose-containing liquid source to generate one or more lactose-containing liquid streams, obtaining a lactose-containing extract from the one or more lactose containing liquid streams by subjecting the one or more lactose-containing liquid streams to filtration to remove cells, proteins, polypeptides, polysaccharides, lipid, ions or salts. The method also includes processing the lactose-containing liquid streams using at least nanofiltration, ultrafiltration and/or centrifugal separation and continuously recycling the lactose-containing extract into the lactose-containing liquid source. US 4 404 038 discloses a process and installation for producing lactose crystals. WO 02/50089 discloses method for purification of lactose. US 2 778 750 relates to ion exchange purification of whey for the preparation of lactose. Y.K.Guu et al., Journal of Food Science, Vol.57, No.3, 1992, pages 735-739 disclose how the nanofiltration concentration affects the efficacy of lactose crystallization.

However, mother liquor typically comprises about 32% by weight total solids (TS). Both mother liquor and wash water also comprise residuals of cells, proteins, polypeptides, lipids ions, salts as well as the remaining lactose. Therefore, when a high fraction of the lactose comprising solution is recycled continuously, the level of impurities increases to an undesirable level. Therefore, typically only a minor part of the lactose from mother liquor and wash water is recovered. The rest is discarded as waste.

The present inventors have now realized that the overall lactose yield can be significantly increased with the same product quality if mother liquor and/or wash water is recycled in a batch process according to the present disclosure.

### SUMMARY OF THE INVENTION

The invention relates to the method for obtaining crystallized lactose, wherein the mother liquor and, when present, wash water are first collected in a stock. Thereafter, mother liquor and, optionally, wash water, are subjected in a batch process to further purification thereby generating a stock of an aqueous solution comprising lactose, which can be further used as an aqueous solution comprising lactose in a lactose crystallization process.

In one embodiment the invention relates to a method for obtaining crystallized lactose (14), comprising: i) in a first crystallization pass (II): subjecting an aqueous solution comprising lactose to a lactose crystallization process (9-12) to obtain crystallized lactose (14) and a mother liquor (15), and, optionally, washing crystallized lactose (11,12) thereby generating one or more batches of wash water comprising lactose (16, 17); collecting mother liquor (15) and, when present, one or more batches of wash water (16,17), thereby obtaining a first stock solution of a first crystallization pass lactose solution; ii) performing in a batch process (19-21) on the first stock solution a concentration pass (IV) comprising demineralizing (20) the first crystallization pass lactose solution to obtain a demineralized first crystallization pass lactose solution, and iii) concentrating (21) the demineralized first crystallization pass lactose solution thereby obtaining a concentrated demineralized first crystallization pass lactose solution; and iv) collecting the concentrated demineralized first crystallization pass lactose solution thereby obtaining a second stock solution (23) comprising the concentrated demineralized first crystallization pass lactose solution; and v) on the second stock solution of the concentrated demineralized first crystallization pass lactose solution perform a second crystallization pass (II) independently of the first crystallization pass.

Another embodiment relates to a method for obtaining crystallized lactose (14), wherein in the second crystallization pass subjecting the second stock solution to the lactose crystallization process (9-12) to obtain a second crystallization pass crystallized lactose (14) and a second crystallization pass mother liquor (15), and, optionally, washing said second crystallization pass crystallized lactose (11, 12) thereby generating one or more batches of wash water comprising lactose (16,17), and, optionally, collecting said second crystallization pass mother liquor (15) and, when present, one or more batches of the second crystallization pass wash water (16, 17) thereby obtaining a first stock of a second crystallization pass lactose solution.

In another embodiment the invention relates to a method for obtaining crystallized lactose (14), wherein the second crystallization pass (II) is repeated at least once as a subsequent crystallization pass (II).

In another embodiment the invention relates to a method for obtaining crystallized lactose (14), wherein the first stock solution of said second crystallization pass is further subjected in a batch process (19-21) to the concentration pass (IV) at least once.

In another embodiment the invention relates to a method for obtaining crystallized lactose (14), wherein crystallization of lactose is allowed to proceed until at least 50% of total lactose present in the aqueous solution comprising lactose or any of the sock of the first, second or subsequent crystallization pass lactose solutions is crystallized.

It is disclosed that a method for obtaining crystallized lactose (14), wherein any first stock of the first, second or subsequent crystallization pass lactose solutions is pasteurized (19) before demineralization (20) in the concentration pass (IV).

In another embodiment the invention relates to a method for obtaining crystallized lactose (14), wherein the first stock solution of the demineralized first crystallization pass or second crystallization pass lactose solution, is collected in a collection tank (22) prior to undergoing further concentration (21) and crystallization in the second or subsequent crystallization pass (II) to obtain crystallized lactose.

In an embodiment the invention relates to a method for obtaining crystallized lactose (14), wherein at least two batches of the first pass or the second pass lactose solutions are combined in the collection tank (22) prior to undergoing concentration (21) and crystallization in the subsequent crystallization pass (II) to obtain crystallized lactose.

In another embodiment the invention relates to a method for obtaining crystallized lactose (14), wherein aqueous solution comprising lactose is whey or whey permeate (1). In another embodiment the invention relates to a method for obtaining crystallized lactose (14), wherein the demineralization (20) is performed by nanofiltration.

It is also disclosed that a method for obtaining crystallized lactose (14), wherein crystallized lactose is further dried in a post treatment step (III).

In another embodiment the invention relates to a method for obtaining crystallized lactose (14), wherein whey or whey permeate is subjected to a pretreatment step (I) thereby obtaining an aqueous solution comprising lactose.

In another embodiment the invention relates to a method for obtaining crystallized lactose (14), wherein the pretreatment step (I) comprises at least a) purifying whey or whey permeate to remove one or more of protein, polypeptides, polysaccharides or lipids, and b) concentrating the whey or whey permeate.

In another embodiment the invention relates to a method for obtaining crystallized lactose (14), wherein the purification of whey or whey permeate (a) is performed by ultrafiltration.

It is also disclosed a method for obtaining crystallized lactose (14), wherein the concentration (b) is performed by evaporation.

In another embodiment the invention relates to an arrangement (30) for obtaining crystallized lactose (14) from an aqueous solution comprising lactose, the arrangement comprising a lactose crystallization line (33) configured for obtaining crystallized lactose from the aqueous solution comprising lactose as a precipitate (9, 10) and one or more batches of mother liquor (15) and, optionally, one or more batches of wash water (16, 17) from washing and separating the precipitated crystallized lactose (11, 12); wherein the arrangement (30) further comprises a mother liquor recycling line (31) configured for executing in a batch process the concentration pass (IV) on the first stock solution of the first or subsequent lactose crystallization pass, comprising a first collection tank (18), configured for collecting the one or more batches of mother liquor (15) and, optionally, the one or more batches of wash water (16,17) thereby obtaining a first stock of the first crystallization pass lactose solution, and accumulating it in a stock, a demineralization unit (34), configured for demineralizing (20) the first stock the first crystallization pass lactose solution to obtain a demineralized first crystallization pass lactose solution; a concentration unit (36), configured for concentrating (21) the demineralized first crystallization pass lactose solution to obtain a concentrated demineralized first crystallization pass lactose solution, a third collection tank configured for collecting the concentrated demineralized first crystallization pass lactose solution; at least one pump (37) for pumping the aqueous solution comprising lactose through the arrangement (30) and a controller (38) configured for executing a method according to any of the previous embodiments of the invention, wherein the first collection tank (18), the demineralization unit (34), the concentration unit (36) and the third collection tank (23) are arranged sequentially in a flow path on the mother liquor recycling line (31), permitting the first stock solution of the first, second or subsequent crystallization pass lactose solution to traverse the mother liquor recycling line (31) in a batch process, thereby accumulating the second stock of the concentrated demineralized first, second or subsequent crystallization pass lactose solution in an amount sufficient for use in a subsequent lactose crystallization pass (II).

In another embodiment the invention relates to an arrangement (30), wherein the mother liquor recycling line (31) further comprises a pasteurization unit (35), configured for pasteurizing (19) the first stock solution of the first or subsequent crystallization pass; wherein the pasteurization unit (35) is located after the first collection tank (18) on the flow path.

In another embodiment the invention relates to an arrangement (30), wherein the mother liquor recycling line (31) further comprises second collection tank (22), configured for receiving and accumulating the demineralized first crystallization pass lactose solution prior to transferring the concentrated demineralized first crystallization pass lactose solution to the lactose crystallization line (33).

In another embodiment the invention relates to an arrangement (30), wherein the controller (38) is configured to allow crystallization of lactose until at least 50% of total lactose present in the aqueous solution comprising lactose is crystallized.

To those skilled in the art to which the invention relates, many changes in construction and differing embodiments and applications of the invention will suggest themselves without departing from the scope of the invention as defined in the appended claims. The disclosures and the descriptions herein are purely illustrative and are not intended to be in any sense limiting.

It is the aim of the present invention to provide a method for obtaining crystallized lactose and the equipment configured for executing the method. The method comprises obtaining initially, from a lactose crystallization process, mother liquor and wash water generated in that process. The obtained mother liquor and wash water are collected and not discarded. Thereafter, the collected mother liquor and wash water are subjected to purification and concentration in a batch process contrary to the known continuous processes, performed independently of the lactose crystallization process. Aqueous solutions comprising lactose obtained as a result of the independent purification and concentration process accordingly, comprise reduced amounts of the impurities usually comprised in the by-products of the lactose crystallization process. The aqueous solution comprising lactose can then be collected and re-used as a lactose source in a lactose crystallization process, either on the same process equipment which supplied the original batch or on separate equipment.

### BRIEF DESCRIPTION OF THE DRAWINGS

An exemplary embodiment of the present invention is illustrated by way of example in the accompanying drawings in which like reference numbers indicate the same or similar elements.

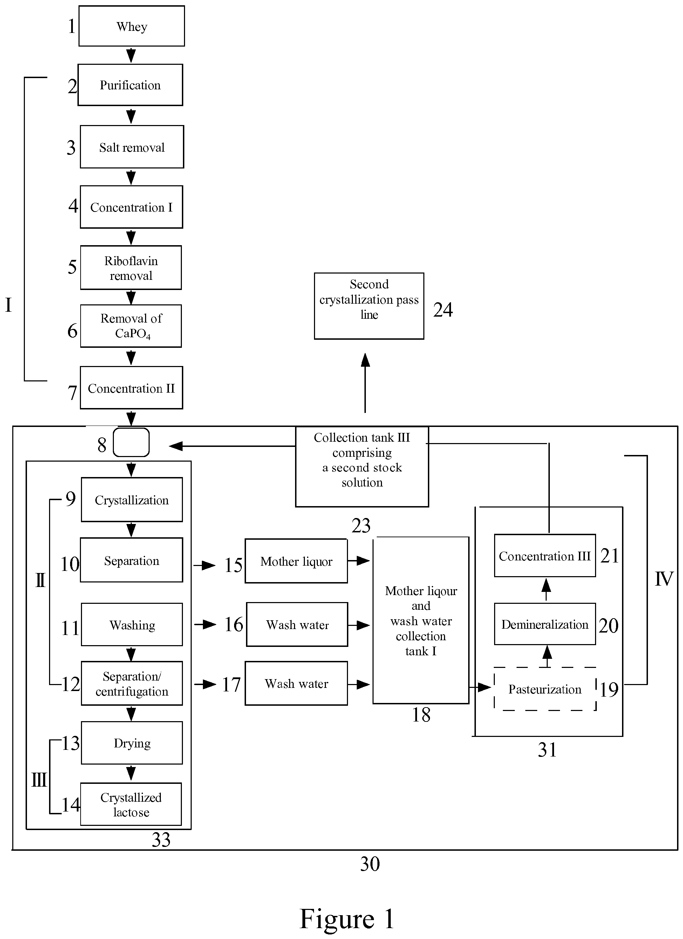

Figure 1: Flow diagram of lactose crystallization starting from whey or whey permeate according to one of the embodiments of the invention, wherein a first stock of the concentrated demineralized first pass lactose solution and/or any second or subsequent pass lactose solution re-enters the concentration step (IV) in a batch process as disclosed herein.

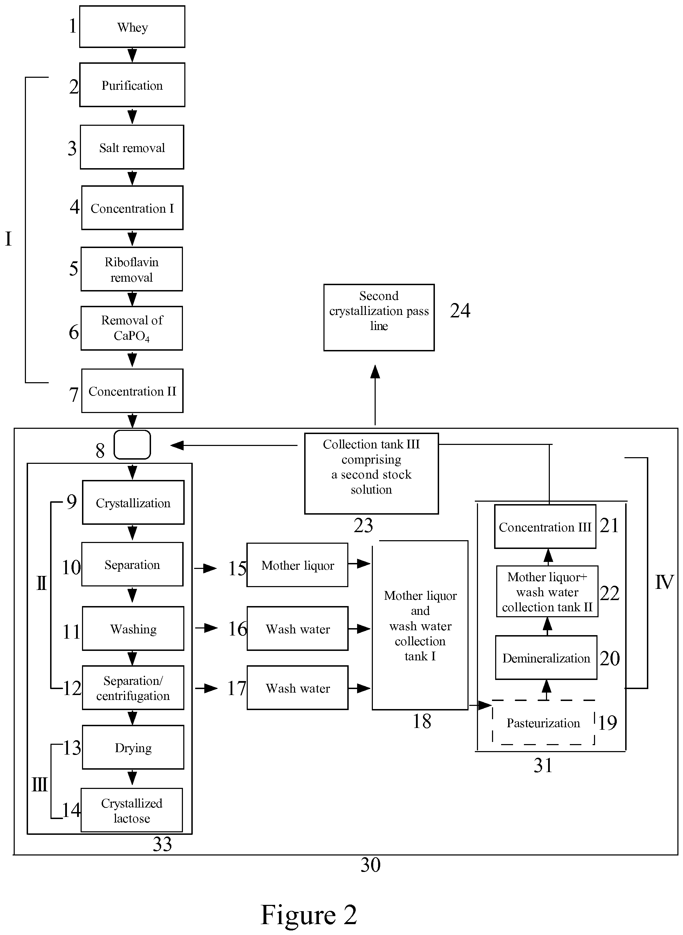

Figure 2: Flow diagram of lactose crystallization starting from whey or whey permeate according to one of the embodiments of the invention, wherein the second stock of the concentrated demineralized first concentration pass lactose solution and/or second or subsequent pass lactose solutions re-enters the concentration step (IV) in a batch process, wherein the first concentration pass lactose solution and/or the second concentration pass lactose solution is gathered in a collection tank prior to concentrating.

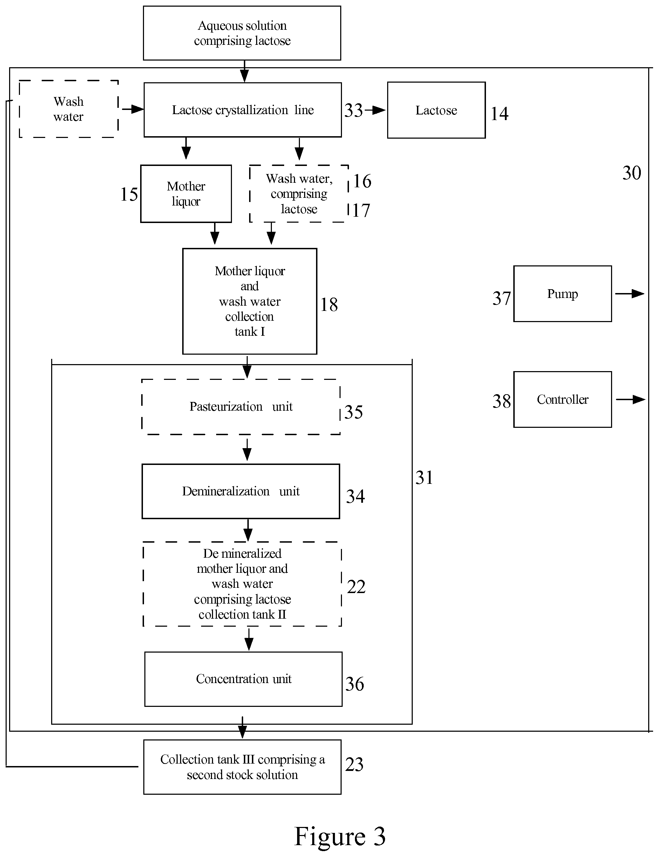

Figure 3: An arrangement for executing the method according to the invention.

### DETAILED DESCRIPTION OF THE INVENTION

The method for obtaining a solid crystallized lactose product from an aqueous solution comprising lactose is described below with reference to the drawings, Figures 1, 2 and 3. The processes and the arrangement described in the flow-diagrams are meant as an illustration of the inventive concept, and the skilled person will be able to make modifications thereof, following the content of the present disclosure, without departing from the disclosed inventive concept.

The present invention relates to a method for obtaining crystallized lactose (14), comprising: i) in a first crystallization pass (II) subjecting an aqueous solution comprising lactose to a lactose crystallization process (9-12) to obtain crystallized lactose (14) and a mother liquor (15), and, optionally, washing said crystallized lactose (11,12) thereby generating one or more batches of wash water comprising lactose (16,17), collecting the mother liquor (15) and, when present the one or more batches of the wash water (16, 17) thereby obtaining a first stock solution of a first crystallization pass lactose solution; ii) performing in a batch process (19-21) on the first stock solution a concentration pass (IV) comprising demineralizing (20) the first crystallization pass lactose solution to obtain a demineralized first crystallization pass lactose solution, and iii) concentrating (21) the demineralized first crystallization pass lactose solution thereby obtaining a concentrated demineralized first crystallization pass lactose solution; and iv) collecting the concentrated demineralized first crystallization pass lactose solution thereby obtaining a second stock solution comprising the concentrated demineralized first crystallization pass lactose solution; and v) on the second stock solution perform a second crystallization pass (II) independently from the first crystallization pass (II).

Crystallized lactose in the context of the present invention preferably means alpha-lactose monohydrate. Aqueous solutions comprising lactose can be whey, whey permeate, or pre-treated whey permeate as detailed further below.

In the art it is known to crystallize lactose from aqueous solutions in a various manners. For example, lactose can be crystallized at room temperature by evaporation of water or by heating and subsequent cooling of the crystallized lactose. Any suitable method for lactose crystallization can be used without departing from the scope of the invention, since the invention is primarily concerned with the ways of recycling the lactose comprising solutions, in particular mother liquor and /or wash water.

In industry, there is a constant demand for high yield lactose crystallization processes. In the improved process of the invention the second stock solution comprising lactose is recovered in a batch process and not continuously.

Since mother liquor as a rule comprises significant quantities of protein, minerals and minerals, impurities accumulates in the prior art solutions and precipitates of crystallized lactose thereby decreasing purity in the final product. The volumes of the mother liquor and wash water are significantly smaller and are constantly fed into the equipment during the continuous process, thereby re-entering not only the remaining lactose but also impurities into the lactose crystallization line.

Therefore, any continuous method of recycling of mother liquor and/or wash water is not optimal, especially in preparation of lactose for the uses wherein high purity is desirable, such as infant formula.

In accordance with the invention, it is advantageous to collect all mother liquor and/or wash water as a stock. A first stock in the context of present disclosure means that the mother liquor and the wash water are taken out of the lactose crystallization process and collected in a suitable container (for example a mother liquor and wash water collection tank I) as a first stock solution comprising lactose. Thereby all mother liquor and wash water obtained in crystallization pass (II) is collected as a stock of the first crystallization pass lactose solution and reused in a batch process as the second stock of the first and/or second crystallization pass lactose solution back to the subsequent crystallization process (II).

Traditionally a yield of up to 69-70% can be obtained by single pass lactose crystallization process without recirculation of mother liquor or wash water.

In a prior art, this amount has been increased by circulating wash water from the sieve back into the process, however, to avoid build-up of impurities only a relatively small part of the lactose in the mother liquor and wash water can be circulated continuously back into the process. The rest has been discarded as waste.

According to the present invention preferably all mother liquor and optionally wash water is collected to obtain a first stock of the first crystallization pass and/or second or subsequent crystallization pass lactose solution, purified, collected as a second stock and returned to the crystallization process, for example by running on the same process equipment as the first crystallization pass (II). When a concentration pass (IV) and a subsequent crystallization pass (II) are used, it is possible to increase the yield significantly with the same product quality.

As detailed according to the present invention the second crystallization pass lactose solution is further subjected to a concentration pass (IV) and a subsequent crystallization pass (II) at least once.

A second stock lactose solution can be accumulated from any number of the subsequent concentration passes (IV) until the desired volume is reached. The volume shall be sufficient for running on the designated equipment and is not limiting on the present invention.

Once the first stock solution of the first crystallization pass is demineralized and concentrated, it is suitable for re-entering the crystallization process, which, according to the present disclosure, is performed after the demineralized and concentrated lactose solution is collected as a second stock solution.

According to the invention more than 70%, preferably more than 75%, more preferably more than 80%, more preferably more than 85%, more preferably more than 90%, more preferably more than 95%, of the lactose present in the first stock solution of the first crystallization pass lactose solution can be recovered following the method of the invention. By performing the method of the invention little or no lactose is lost as waste. It is possible according to the principle of the invention to collect mother liquor and wash water from further subsequent crystallization passes to obtain further stock solutions comprising lactose after repeating the concentration pass (IV) and the crystallization pass (II).

As detailed a method, wherein the first crystallization pass lactose solution or any of the subsequent crystallization pass lactose solutions obtained during one or more repeated concentration passes (IV) are advantageously collected as a second stock solution and accumulated in a tank for mother liquor and wash water (18) prior to each crystallization pass (II) executed as a batch process is described.

As further detailed a method, wherein crystallization of lactose is allowed to proceed until at least 50% of total lactose present in the aqueous solution comprising lactose or any of the first or subsequent crystallization pass lactose solution is crystallized. Preferably, crystallization is permitted to proceed until at least 55%, at least 60%, at least 65% or at least 70% of total lactose present in the aqueous solution comprising lactose or any of the first or subsequent crystallization pass lactose solution are subjected crystallization. Further, preferably, crystallization is terminated before at least 85%, preferably at least 80% of total lactose present in the aqueous solution comprising lactose or any of the first or subsequent crystallization pass lactose solution is crystallized.

Crystallization of aqueous solutions comprising lactose in crystallization tanks as detailed in the prior art is a slow process, typically based on cooling de-colored and concentrated whey permeate by an initial rapid cooling followed by subsequent slow cooling at about 1°C per hour.

In the prior art in order to maximize the yield of lactose from crystallization, a maximal time is allocated to the crystallization step, typically in excess of 20 hours. A problem of the prior art crystallization methods is that that a part of the lactose initially present which after crystallization is still in solution (solvated) or present as very small crystals (suspended) will not be recovered in the subsequent isolation of lactose crystals under industrial conditions, and will therefore go to waste with mother liquor and wash water.

The present invention is highly efficient in regaining and obtaining by further crystallization a large part of this lactose, because the second stock of the purified and concentrated first crystallization pass and/or subsequent crystallization pass lactose solution will comprise not only solvated but also suspended lactose, which is recycled into the crystallization process as a batch after concentration to sufficiently high lactose concentrations for efficient crystal growth. Such concentration is possible only when sufficient amounts of mother liquor and wash water are collected, and according to the present disclosure it can be done, for example, in a mother liquor and wash water collection tank (18).

The method suggested by the present invention allows for the possibility of differentiating the crystallization of each subsequent crystallization pass (II). Since most of the lactose from the second stock of the first crystallization pass lactose solution is recovered in a subsequent crystallization pass (II), it is no longer so important to maximize the yield of the first crystallization pass (II). This means that it is possible to shorten the crystallization time for the first crystallization pass (II). It can for example allow for using the remaining time for the subsequent crystallization pass (II) so that no extra capacity on crystallization tanks is required even when capacity for second pass is used while still resulting in a higher overall yield. If extra capacity for crystallization is reserved for the subsequent pass, then this will increase the overall yield even further.

The subsequent crystallization pass (II) is usually performed on the same equipment as the first crystallization pass (II). This is a further advantage of the method because the lactose yield can be significantly increased without providing any further crystallization tanks or additional equipment. However, a second dedicated recycling line (24) is equally useful, where high throughput rather than equipment cost is in focus.

The mixture obtained during the concentration pass (IV) is collected in a collection tank II (23) comprising a second stock solution. Further, the mixture enters the subsequent crystallization pass independently from the mixture obtained during the first crystallization pass (II). The entering point has a connection (8); connection can be for example a valve, but it should be understood that any suitable connection is possible and the type of connection is not in any way limiting for the invention.

According to suitable methods of the prior art of lactose crystallization and repeated here by way of example, permeate concentrated in an evaporator in step (9) can be cooled to at least 55°C, more preferably 60°C, in a flash cooler built together with the evaporator and kept at this temperature during filling of the crystallization tank(s). When a tank is full, the temperature is lowered by approximately 3°C per hour, until a temperature of approximately 30°C is reached. The cooling speed is then regulated to approximately 1.5°C per hour, until the temperature reaches 15°C. If filling a tank through the bottom, the tank temperature is adjusted to approximately the temperature of the liquid exiting the flash. When cooling starts, cooling e.g. by tower water is used to bring the temperature down as far as possible. Other cooling techniques are equally suitable. As is known in the art, this will result in precipitation of alpha-lactose monohydrate at a good product quality.

The cooling temperature as well as the cooling time can be changed if needed, as it is known to the skilled person.

Mother liquor (15), as known, is that part of a solution, which is left over after crystallization. Typically, mother liquor comprises solvated lactose and a number of impurities. Therefore, continuously re-circulating the mother liquor into the process typically results in an ever-increasing amount of impurities. Also, wash water used to wash crystallized lactose will comprise both lactose and impurities, presenting the same problems vis-a-vis recycling as the mother liquor.

To overcome the above limitations it is suggested by the present inventors that the mother liquor (15) and one or more batches of the wash water (16,17) are collected to obtain a stock solution comprising lactose thereby generating the first stock solution of the first crystallization pass lactose solution. And on the first stock solution to perform in a batch process (18-21)a concentration pass (IV) comprising demineralizing (20)the first crystallization pass lactose solution to obtain a demineralized first and/or second or subsequent crystallization pass lactose solution, followed by concentration (21) of the demineralized first and/or second or subsequent concentration pass lactose solution thereby obtaining a concentrated demineralized first and/or subsequent crystallization pass lactose solution comprising lactose.

It is exemplified in the drawings (Fig. 1 and 2) that concentration (21) of the demineralized aqueous solution comprising lactose is carried out on dedicated process equipment during the batch executed concentration pass (IV); but it should be understood that it can be carried out on equipment for concentration of aqueous solution comprising lactose during pre-treatment step (I), or any other suitable equipment. When this is done, the primary /first crystallization pass process is suspended.

A batch process in the context of the application means that the mother liquor and, optionally, the wash water do not enter the lactose crystallization process continuously but rather are collected to obtain a first stock solution comprising lactose and subjected in a batch process to the steps of extra demineralization (20) and crystallization (21) followed by further collecting as a second stock solution prior to re-entering the lactose crystallization process independently of the first crystallization process.

Crystallized lactose after crystallization (9) can e.g. in one embodiment, initially be separated in a decanter (10), wherein the separated mother liquor (15) contains most of the minerals. The mother liquor (15) will typically be collected in a mother liquor and wash water collecting tank I (18) prior to mixing the mother liquor and the wash water (16, 17)as a first stock solution.

As exemplified, it is possible to wash the crystals obtained from the crystallization step (9) in a decanter.

Optionally, further washing (12) may be done in a tank connected to a decanter. In this manner wash water (16) may also be collected in the mother liquor + wash water collecting tank (18), thereby directly mixing these liquids.

In the method, the crystals from the tank and decanter (11) are separated in a separation step (12), typically by sieve centrifugation optionally further comprising a tank in front, and the wash water (17) formed during this process can then be collected in a mother liquor + wash water collection tank (18).

Concentration of the demineralized first crystallization pass and/or second or subsequent pass lactose solution can be performed by several techniques, for example by evaporating aqueous component as it is known in the art.

As detailed, a method, wherein the first crystallization pass or the second crystallization pass lactose solution is pasteurized (19) before demineralization (20) in the concentration pass (IV) is disclosed.

Pasteurization methods known from the state of the art are suitable for use in the method according to the invention. According to one of the embodiments of the present invention, the first crystallization pass or the second or subsequent crystallization pass lactose solution can be heat treated and cooled before it is demineralized (20).

The pasteurization (19) can for example take place in a regenerative plate heat exchanger by indirect heating with product and with hot water heated by steam. The mix of the mother liquor and the wash water is then kept at approximately between 70°C and 80°C, more preferably for 71°C and 69°C, more preferably 72°C and 68°C, more preferably 73°C and 76°C, more preferably 75°C in a holding cell, for the time sufficient to effectuate the pasteurization. During the pasteurization any remaining small lactose crystals will be dissolved.

After pasteurization (19) the first crystallization pass or the second or subsequent crystallization pass lactose solution is cooled, for example, in the heat exchanger.

As detailed a method, wherein the demineralized first crystallization pass or the second or subsequent crystallization pass lactose solution, is collected in a collection tank (22) prior to undergoing concentration (21) and crystallization in the further subsequent crystallization pass (II) to obtain crystallized lactose is described.

Collecting the demineralized first crystallization pass and/or the second or subsequent crystallization pass lactose solution in the collection tank as a stock allows for operating both the first crystallization pass and (II) and any of the subsequent crystallization passed (II) on the same production line.

Since with each batch pass of the crystallization pass (IV) the amount of water is reduced by concentration; it will often be advantageous to combine third and higher pass lactose solutions from several process lines or process runs in a dedicated, separate, collection tank (22) until a sufficient volume of third or higher pass lactose solutions has been collected; which sufficient volume allows for efficient use of the crystallization tanks at hand in the production facilities where the method of the invention has been implemented.

Accordingly, in an embodiment the present invention comprises combining at least two batches of the first crystallization pass or the second or subsequent crystallization pass lactose solutions in the collection tank (22) prior to undergoing concentration (21) and crystallization in the further subsequent crystallization pass (II) to obtain crystallized lactose.

As detailed, a method wherein aqueous solution comprising lactose is whey or whey permeate (1) is described.

Equally suitable is whey from diary processes, for example mozzarella whey, hard cheese whey and semi hard cheese whey. Incoming whey from e.g. cheese fabrication can initially be processed by separate methods as is known in the art and thus not described in this application. Once whey is pre-treated by the techniques known in the art to generate semi-purified whey suitable for filtration, it is suitable for use in the method of present invention.

As detailed a method, wherein the demineralization (20) is performed by nanofiltration is described.

Demineralization is performed in order to remove as much mineral impurities as possible. Numerous techniques are known in the art for removing mineral impurities from aqueous solutions. It is exemplified in the present application but not limiting the use of a nanofiltration membrane for demineralization.

As detailed, a method wherein crystallized lactose is further dried in a post-treatment step (III) is described.

Different techniques known in the art can be used for lactose drying without departing from the scope of the invention. In one embodiment, as an example only, in a post treatment step (III) of lactose obtaining process, the product obtained after washing (10) or optional additional washing (12) is subsequently fed directly into fluid bed dryer (13), wherein the lactose (14) is finally dried and cooled in a fluid bed system (13).

In the examples, the product is fed from a separation (10) and optional washing (11, 12) section to a drying section (13) in the post treatment step (III).

As detailed, a method wherein whey or whey permeate is subjected to a pre-treatment step (I) thereby obtaining an aqueous solution comprising lactose is described.

The pre-treatment step (I) can be performed by using any of the several techniques known in the art. According to one of the embodiments the pre-treatment step (I) comprises at least a) purifying whey or whey permeate to remove one or more of cells, polypeptides, polysaccharides or lipids and/or b) concentrating whey or whey permeate to obtain purified whey permeate.

Optionally, whey permeate can be purified, riboflavin can be removed from the concentrated whey permeate, or permeate can be de-calcinated prior to concentration.

Purification of whey or whey permeate in the pre-treatment step (I)(a) is typically performed by ultrafiltration. The aim of ultrafiltration is to produce purified whey permeate for further processing according to the present invention.

Concentration of the purified permeate in the pre-treatment step (I) can be performed by for example, reverse osmosis or any other method known in the art.

In accordance with one suggested process of the invention as detailed in the exemplary flow-diagram Figure 1, whey or whey permeate (1) is purified (2) and salts are removed by nanofiltration which will also concentrate the lactose (3). If not already concentrated in (2), then it is concentrated to remove surplus water in a permeate concentration step (4). It is generally preferred that concentration is by reverse osmosis, but other methods of concentrating whey permeate are equally useful.

Whey permeate suitable for use in the present invention is typically concentrated to between 10 to 40% dry solids (DS), between 15 to 35% DS, between 17 to 30% DS, preferably between 18 to 25% DS, and most preferably 20% DS. Concentrated solutions comprising lactose are highly viscous and inefficient processes will typically result if concentrated beyond about 40% dry solids.

The plants used in for concentration of permeate are in general intended for continuous operation. The product is pumped to the plant only once and leaves when concentrated to the required solids content. The choice of membrane is based on the pre-treatment history of the incoming UF-permeates and on the capacity composition and quality of the raw materials, as well as the mass balance, as is known in the art.

The permeate obtained in step (4) is usually treated to a riboflavin removal step (5) for the removal of riboflavin present in the whey permeate, typically by passing the concentrated whey permeate through a column comprising active carbon, thereby removing riboflavin by adsorption (de-colouring process). Other methods of removing of riboflavin are equally useful and the present invention is not limited by the manner in which riboflavin is removed.

In a method for obtaining crystallized lactose (14), optional de-calcinating of the protein free whey in a pre-treatment step (I) can be performed by heating and cooling of concentrated protein free whey permeate. It can also be beneficial to adjust the pH of the concentrated solutions. The above techniques are known in the art and not limiting for the invention. During this time most of the calcium is precipitated as calcium phosphate and can be removed from the solution e.g. by clarification.

In a further aspect of the invention there is disclosed an arrangement (30) for obtaining crystallized lactose from an aqueous solution comprising lactose. The arrangement (30) comprises a lactose crystallization line (33) and a mother liquor recycling line (31), configured for executing the method according to any of the earlier described embodiments of the invention. This embodiment is detailed further below with reference to Figures 1, 2 and 3.

As detailed, an arrangement for obtaining crystallized lactose (14) from an aqueous solution comprising lactose, the arrangement comprising: a lactose crystallization line (33) configured for obtaining crystallized lactose from the aqueous solution comprising lactose as a precipitate (9,10) and one or more batches of mother liquor (15) and, optionally, one or more batches of wash water (16,17) from washing and separating said precipitated crystallized lactose (11,12) wherein the arrangement (30) further comprises a mother liquor recycling line (31) configured for executing the concentration pass (IV) on the first stock solution of the first, second or subsequent crystallization pass lactose solution comprising: a first collection tank (18), configured for collecting the one or more batches of mother liquor (15) and, optionally, one or more batches of wash water (16, 17) as a first stock of the first crystallization pass lactose solution; a demineralization unit (34), configured for demineralizing (20) the first stock of the first crystallization pass lactose solution to obtain a demineralized first crystallization pass lactose solution; a concentration unit (36), configured for concentrating (21) the demineralized first crystallization pass lactose solution to obtain a concentrated demineralized first crystallization pass lactose solution, a third collection tank configured for collecting the concentrated demineralized first crystallization pass lactose solution thereby obtaining the second stock of the demineralized first, second or subsequent crystallization pass lactose solution; at least one pump (37) for pumping the aqueous solution comprising lactose through the mother liquor recycling line (31); and a controller (38) configured for executing a method according to any embodiment of the invention, wherein the first collection tank (18), the demineralization unit (34), the concentration unit (36) and the third collection tank (23) are arranged sequentially on a flow path in said mother liquor recycling line (31) permitting the first stock solution of the first or subsequent crystallization pass lactose solution to traverse said mother liquor recycling line (31) in a batch process, thereby accumulating the second stock of the concentrated demineralized first or subsequent crystallization pass lactose solution in the amount sufficient for use in a further subsequent lactose crystallization pass (II).

The arrangement (30) receives an aqueous solution comprising lactose, which can be obtained in any manner used in the art. It can be incoming whey (1) as defined in the Figure 1 and 2; however, the source is not considered limiting on the present invention.

The aqueous solution comprising lactose traverses through the arrangement (30) by a sequence of process units (23, 33, 35, 34, 36,) sequentially placed on a flow path. The flow path fluidly connects the process units in a sequence, e.g. by use of pipes and conducts. The aqueous solution comprising lactose enters at the lactose crystallization unit (33), thereby generating lactose as a precipitate (14) and one or more batches of mother liquor (15), and the one or more batches of wash water (16, 17) to obtain a first or subsequent crystallization pass lactose solution in a first stock.

Further, the mother liquor (15) and, optionally, wash water comprising lactose (16,17) traverses the mother liquor recycling line (31), by a sequence of process units (35, 34, 36), operatively arranged on the flow path. To serve this purpose the flow pass fluidly connects the process units in a sequence, e.g. by use of pipes and conducts. The mother liquor (15) and, optionally, one or more batches of wash water comprising mother liquor (16, 17) can be pumped through the mother liquor recycling line (31) from its entry point using at least one pump (37). The at least one pump will be installed in the arrangement (30) according to the specific requirements of the lactose crystallization line (33) and /or mother liquor recycling line (31) of the invention to assure the efficient flow of the aqueous solution comprising lactose through the arrangement.

The sequence of process units (23, 35, 34, and 36) are operatively arranged on the flow path in such a way that matter may enter or exit the flow path without the overall flow direction of the solution thereby being changed. A controller configured for executing the process according to any of the embodiments of the present invention is further comprised in the lactose crystallization line.

With reference to Figures 1 and 3, an arrangement (30) for obtaining crystallized lactose (14) from an aqueous solution comprising lactose comprises a first collection tank (18), configured for collecting the one or more batches of mother liquor (15) and/or the one or more batches of wash water comprising lactose (16, 17) to obtain the first crystallization pass lactose solution. The first pass lactose solution is further collected and accumulated in the first collection tank (18) as a first stock solution.

The first collection tank (18) is further connected to the demineralization unit (34) configured for demineralizing (20) the first pass lactose solution to obtain a demineralized first crystallization pass lactose solution. Further it is configured for permitting the demineralized first crystallization pass lactose solution to enter the concentration unit (36). The concentration unit (36) is configured to generate the concentrated demineralized first crystallization pass lactose solution, suitable for use in a second or subsequent crystallization pass (II).

The concentration unit (36) will usually be configured for concentrating (21) by evaporation, but the method is not limiting for the invention.

According to another embodiment of the invention the concentration unit (36) can also be configured to concentrate (7) an aqueous solution comprising lactose obtained from whey or whey permeate (1) during pretreatment step (I).

The mother liqueur recycling line (31) is further configured to permit accumulation of the concentrated demineralized first or subsequent crystallization pass lactose solution in an amount sufficient for use in a subsequent lactose crystallization pass (II) in the third tank (23) thereby obtaining a second stock of the demineralized concentrated first of second crystallization pass lactose solution.

As detailed here with reference to Figure 2, the second collection tank (22) optionally can be located on the flow path. The second collection tank (22) is configured for receiving and accumulating demineralized first crystallization pass lactose solution prior to transferring the demineralized lactose solution to the concentration unit (36). The second collection tank (22) permits accumulation of the demineralized solution until it reaches any desired volume before allowing the demineralized first crystallization pass lactose solution exits the storage tank and traverse the lactose crystallization line (33) of the invention. The desired volume can vary depending on particulars of various processes and is not limiting for the invention and can be easily set programmed into the controller (38). The advantage of the mother liquor recycling line (31) configured for executing the batch method of the invention is that the first crystallization pass lactose can be collected to the desired amount and subjected to demineralization in order to clear it from impurities and render it suitable for the subsequent crystallization pass of the invention.

Further with reference to Figure 3, the concentration unit (36) generates the aqueous solution comprising lactose, thereby making it suitable for entering lactose crystallization unit (33). The concentration unit (36) can be connected to the lactose crystallization unit (33) directly or through a number of additional units suitable for executing all embodiments of the method of the invention; the additional units are not detailed herein since they are not limiting to the invention. The controller (38) is further configured to allow for repeating of the concentration pass (IV) and subsequent crystallization pass (II) at least once.

As detailed below a pasteurization unit (35), configured for pasteurizing (19) the first crystallization pass lactose solution is located on the mother liquor recycling line (31) after first collection tank (18); preferably but not necessarily directly after first collection tank (18).

As detailed below the mother liquor recycling line (31) can further comprise second collection tank (22), configured for receiving and accumulating a demineralized first, second or subsequent crystallization pass lactose solution prior to transferring the demineralized first or second or subsequent crystallization pass lactose solution to the concentration unit (36). The second collection tank (22) is configured for receiving and accumulating the demineralized first concentration pass lactose solution until the desired volume. The controller is configured to control the accumulation until the desired volume and further entering the concentration tank when the desired volume is reached. Another advantage of the arrangement of the invention that it allows for accumulating of the concentrated demineralized first crystallization pass lactose solution to the volume suitable for us in the subsequent lactose crystallization pass (II) as a stock.

Preferably, the controller (37) is configured to allow crystallization of lactose until at least 50% of total lactose present in the aqueous solution comprising lactose is crystallized.

### EXAMPLES

### Example 1

0.075% NaOH was added to the initial pre-treatment step (I) wherein it has been heated to 75°C for 1 hour, clarified and 0.04% HCl was added. Product was evaporated (6) to 60% DM and crystalized (9), separated (10), washed (11) and dried (13).

All wash water (15, 16) and mother liquor (14) was collected for the concentration pass (IV). From this step (IV) the monovalent salts was removed in NF plant (20). The retentate was then heated to 75°C for 1 hour (5), clarified (6), and 0.04% HCl was added. The obtained product was then concentrated to 64% DM (7), crystallized (9), separated (10), washed (11), and dried (13). The total crystallization time was about 23 hours.
a. Results of product was 0.13% sulfated ash in the first crystallization pass (II) product and 0.11% sulfated ash in a subsequent crystallization pass (II) product.
b. Yield of the first crystallization pass (II) was 69% measured on anhydrous lactose and yield of the subsequent crystallization pass (II) was 15% measured on anhydrous lactose, giving a total yield of 84% measured on anhydrous lactose. This yield includes all losses in process.

### Example 2

0.065% NaOH was added to the initial pre-treatment step (I) followed by heating to 76°C for 1 hour (4), clarified (6), and 0.04% HCl was added. Product was evaporated to 60% DM (7) and crystalized (9), separated (10), washed (11) and dried (13). All wash water (16, 17) and mother liquor (15) was collected for the concentration pass (IV). From this concentration pass (IV) the monovalent salts was removed in NF plant (20). The obtained product then entered the subsequent crystallization pass (II). The retentate was then heated to 75°C for 1 hour (5), clarified (6), and 0.03% HCl was added. Subsequent crystallization pass product (II) was then concentrated to 64% DM (7), crystallized (8), separated (10), washed (11), and dried (13). The total crystallization time was about 23 hours.
a. Results of product was 0.11% sulfated ash in the first crystallization pass (II) product and 0.12% sulfated ash in the subsequent crystallization pass (II) product.
b. Yield of the first crystallization pass (II) was 68% measured on anhydrous lactose and yield of the subsequent crystallization pass (II) was 14% measured on anhydrous lactose, giving a total yield of 82% measured on anhydrous lactose. This yield includes all losses in process.

Using the known methods of the prior art and permitting the lactose comprising solution to crystallize for the same period as in the above examples (everything else constant) will yield below 75% lactose measured on anhydrous lactose.

### CLOSING COMMENTS

The term "comprising" as used in the claims does not exclude other elements or steps. The term "a" or "an" as a used in the claims does not exclude a plurality. And although the present invention has been described in detail for purpose of illustration, it is understood that such detail is solely for that purpose, and variations can be made therein by those skilled in the art without departing from the scope of the invention.

## Claims

1. A method for obtaining crystallized lactose (14), comprising:
i) in a first crystallization pass (II):
- subjecting an aqueous solution comprising lactose to a lactose crystallization process (9-12) to obtain crystallized lactose (14) and a mother liquor (15), and,
- optionally, washing said crystallized lactose (11,12) thereby generating one or more batches of wash water comprising lactose (16,17),
- collecting said mother liquor (15) and, when present, said one or more batches of said wash water (16, 17) thereby obtaining a first stock of a first crystallization pass lactose solution;
ii) performing in a batch process (19-21) on said first stock solution a concentration pass (IV) comprising
- demineralizing (20) said first crystallization pass lactose solution to obtain a demineralized first crystallization pass lactose solution, and
iii) concentrating (21) said demineralized first pass lactose solution thereby obtaining a concentrated demineralized first crystallization pass lactose solution;
iv) collecting said concentrated demineralized first crystallization pass lactose solution thereby obtaining a second stock solution comprising said concentrated demineralized first concentration pass lactose solution; and
v) on said second stock solution perform a second crystallization pass (II) independently of said first crystallization pass.

2. A method according to claim 1, comprising in said second crystallization pass (II)
- subjecting said second stock solution to said lactose crystallization process (9-12) to obtain a second pass crystallized lactose (14) and a second pass mother liquor (15),
- optionally, washing said second pass crystallized lactose (11,12), thereby generating one or more batches of wash water comprising lactose (16,17),
- optionally, collecting said second crystallization pass mother liquor (15) and, when present, said one or more batches of wash water (16, 17), thereby obtaining a first stock of a second crystallization pass lactose solution.

3. A method according to any of claims 1 or 2, wherein said second crystallization pass (II) is repeated at least once as a subsequent crystallization pass (II).

4. A method according to any of claims 1 to 3, wherein said first stock solution of said second crystallization pass (II) is further subjected in a batch process (19-21) to said concentration pass (IV) at least once.

5. A method according to any of claims 1 to 4, wherein crystallization of lactose (8) is allowed to proceed until at least 50% of total lactose present in said aqueous solution comprising lactose or any of said stock of said first, second or subsequent passes lactose solutions is crystallized.

6. A method according to any of claims 1 to 5, wherein said first stock of said demineralized first, second or subsequent pass lactose solution, is collected in a collection tank (22) prior to undergoing further concentration (21) and crystallization in said second or subsequent crystallization pass (II) to obtain crystallized lactose.

7. A method according to any of the claims 1 to 6, wherein at least two batches of said first stock of said first pass or said second pass lactose solutions are combined in said collection tank (22) prior to undergoing in a batch process said concentration (21) and crystallization in said subsequent crystallization pass (II) to obtain crystallized lactose.

8. A method according to any of claims 1 to 7, wherein aqueous solution comprising lactose is whey or whey permeate (1).

9. A method according to any of claims 1 to 8, wherein said demineralization (20) is performed by nanofiltration.

10. A method according to any of claims 1 to 9, wherein whey or whey permeate is subjected to a pretreatment step (I) thereby obtaining an aqueous solution comprising lactose, wherein said pretreatment step (I) comprises:
a) purifying whey or whey permeate to remove one or more of protein, polypeptides, polysaccharides, lipids, or riboflavin, and
b) concentrating said purified whey or whey permeate.

11. A method according to claims 12 or 13, wherein said purification of whey or whey permeate (a) is performed by ultrafiltration.

12. An arrangement (30) for obtaining crystallized lactose (14) from an aqueous solution comprising lactose, said arrangement comprising:
- a lactose crystallization line (33) configured for obtaining crystallized lactose from said aqueous solution comprising lactose as a precipitate (9,10) and one or more batches of mother liquor (15) and, optionally, one or more batches of wash water (16,17) from washing and separating said precipitated crystallized lactose (11,12);
**characterized in that** said arrangement (30) further comprises:
- a mother liquor recycling line (31) configured for executing said concentration pass (IV) on a first stock solution of said first, second and/or subsequent lactose crystallization pass comprising:
- a first collection tank (18), configured for collecting said one or more batches of mother liquor (15) and, optionally, said one or more batches of wash water (16, 17) as said first stock of said first crystallization pass lactose solution;
- a demineralization unit (34), configured for demineralizing (20) said first stock of said first crystallization pass lactose solution to obtain a demineralized first crystallization pass lactose solution;
- a concentration unit (36), configured for concentrating (21) said demineralized first crystallization pass lactose solution to obtain a concentrated demineralized first crystallization pass lactose solution,
- a third collection tank configured for collecting said concentrated demineralized first pass lactose solution thereby obtaining a second stock of said demineralized first, second and/or subsequent crystallization pass lactose solution;
at least one pump (37) for pumping said aqueous solution comprising lactose through said mother liquor recycling line (31); and
a controller (38) configured for executing a method according to any of the claims 1 to 15,
wherein said first collection tank (18), said demineralization unit (34), said concentration unit (36) and said third collection tank are arranged sequentially on a flow path in said mother liquor recycling line (31) for permitting a first stock of said first or subsequent lactose crystallization pass lactose solution to traverse said mother liquor recycling line (31) in a batch process, thereby accumulating a second stock of said concentrated demineralized first or subsequent crystallization pass lactose solution in an amount sufficient for use in a further subsequent lactose crystallization pass (II).

13. An arrangement according to claim 12, wherein said mother liquor recycling line (31) further comprises a pasteurization unit (35), configured for pasteurizing (19) said first stock of said first crystallization pass lactose solution; wherein said pasteurization unit (35) is located after said first collection tank (18) on said flow path.

14. An arrangement according to any of claims 12 or 13, wherein said mother liquor recycling line (31) further comprises second collection tank (22), configured for receiving and accumulating demineralized first crystallization pass lactose solution prior to transferring said concentrated demineralized first crystallization pass lactose solution to said lactose crystallization line (33).

15. An arrangement according to any of claims 12 to 14, wherein said controller (38) is configured to allow crystallization of lactose (8) until at least 50% of total lactose present in said aqueous solution comprising lactose is crystallized.

## Patentansprüche

1. Verfahren zum Erhalten von kristallisierter Laktose (14), umfassend:
i) in einem ersten Kristallisationsdurchgang (II):
- Unterwerfen einer wässrigen Lösung, die Laktose umfasst, einem Laktosekristallisationsverfahren (9-12), um kristallisierte Laktose (14) und eine Mutterlauge (15) zu erhalten, und,
gegebenenfalls, Waschen der kristallisierten Laktose (11, 12), wodurch eine oder mehrere Chargen Waschwasser erzeugt werden, die Laktose (16, 17) umfassen,
- Sammeln der Mutterlauge (15) und, wenn vorhanden, der einen oder mehreren Chargen des Waschwassers (16, 17), wodurch ein erster Vorrat einer Erst-Kristallisationsdurchgangs-Laktoselösung erhalten wird;
ii) Durchführen eines Konzentrationsdurchgangs (IV) in einem Batch-Prozess (19-21) mit der ersten Vorratslösung, umfassend
- Entmineralisieren (20) der Erst-Kristallisationsdurchgangs-Laktoselösung, um eine entmineralisierte Erst-Kristallisationsdurchgangs-Laktoselösung zu erhalten, und
iii) Konzentrieren (21) der entmineralisierten ersten Erst-Kristallisationsdurchgangs-Laktoselösung, wodurch eine konzentrierte entmineralisierte Erst-Kristallisationsdurchgangs-Laktoselösung erhalten wird;
iv) Sammeln der konzentrierten entmineralisierten Erst-Kristallisationsdurchgangs-Laktoselösung, wodurch eine zweite Vorratslösung erhalten wird, die die konzentrierte entmineralisierte Erst-Kristallisationsdurchgangs-Laktoselösung umfasst; und
v) mit der zweiten Vorratslösung einen Zweit-Kristallisationsdurchgang (II) unabhängig vom Erst-Kristallisationsdurchgang durchführen.

2. Verfahren nach Anspruch 1, das in dem zweiten Kristallisationsdurchgang (II) Folgendes umfasst:
- Unterwerfen der zweiten Vorratslösung dem Laktosekristallisationsverfahren (9-12), um eine kristallisierte Zweit-Durchgangs-Laktose (14) und eine Zweitdurchgangs-Mutterlauge (15) zu erhalten,
- gegebenenfalls, Waschen der kristallisierten Zweitdurchgangs-Laktose (11, 12), wodurch eine oder mehrere Chargen von Waschwasser erzeugt werden, die Laktose (16, 17) umfassen,
- gegebenenfalls, Sammeln der Zweitdurchgangs-Kristallisationsmutterlauge (15) und, wenn vorhanden, der einen oder mehreren Chargen Waschwasser (16, 17), wodurch ein erster Vorrat einer Zweitdurchgangs-Kristallisations-Laktoselösung erhalten wird.

3. Verfahren nach einem der Ansprüche 1 oder 2, wobei der zweite Kristallisationsdurchgang (II) mindestens einmal als nachfolgender Kristallisationsdurchgang (II) wiederholt wird.

4. Verfahren nach einem der Ansprüche 1 bis 3, wobei die erste Vorratslösung des Zweit-Kristallisationsdurchgangs (II) ferner in einem Batch-Prozess (19-21) mindestens einmal dem Konzentrationsdurchgang (IV) unterworfen wird.

5. Verfahren nach einem der Ansprüche 1 bis 4, wobei die Kristallisation von Laktose (8) so lange fortgesetzt werden darf, bis mindestens 50 % der gesamten Laktose, die in der wässrigen Lösung umfassend Laktose oder einen des Vorrats der Erst-, Zweit- oder Folgedurchgangs-Laktoselösungen vorhanden sind, kristallisiert werden.

6. Verfahren nach einem der Ansprüche 1 bis 5, wobei der erste Vorrat der entmineralisierten Erst-, Zweit- oder Folgedurchgangs-Laktoselösung in einem Sammelbehälter (22) gesammelt wird, bevor eine weitere Konzentration (21) und Kristallisation in dem Zweit- oder Folge-Kristallisationsdurchgang (II) erfolgt, um kristallisierte Laktose zu erhalten.

7. Verfahren nach einem der Ansprüche 1 bis 6, wobei mindestens zwei Chargen des ersten Vorrats von Erstdurchgangs- oder Zweitdurchgangs-Laktoselösungen in dem Sammelbehälter (22) kombiniert werden, bevor die Konzentration (21) und die Kristallisation in dem nachfolgenden Kristallisationsdurchgang (II) in einem Batchverfahren durchgeführt werden, um kristallisierte Laktose zu erhalten.

8. Verfahren nach einem der Ansprüche 1 bis 7, wobei die wässrige Lösung, die Laktose umfasst, Molke oder Molkepermeat (1) ist.

9. Verfahren nach einem der Ansprüche 1 bis 8, wobei die Entmineralisierung (20) durch Nanofiltration durchgeführt wird.

10. Verfahren nach einem der Ansprüche 1 bis 9, wobei Molke oder Molkepermeat einem Vorbehandlungsschritt (I) unterzogen wird, wodurch eine wässrige Lösung erhalten wird, wobei der Vorbehandlungsschritt (I) Folgendes umfasst:
a) Reinigen von Molke oder Molkepermeat, um eines oder mehrere von Proteinen, Polypeptiden, Polysacchariden, Lipiden oder Riboflavin zu entfernen, und
b) Konzentrieren der gereinigten Molke oder des Molkepermeats.

11. Verfahren nach den Ansprüchen 12 oder 13, wobei die Reinigung von Molke oder Molkepermeat (a) durch Ultrafiltration durchgeführt wird.

12. Anordnung (30) zum Erhalten von kristallisierter Laktose (14) aus einer wässrigen Lösung, die Laktose umfasst, wobei die Anordnung Folgendes umfasst:
- eine Laktosekristallisationslinie (33), die konfiguriert ist, um kristallisierte Laktose aus der wässrigen Lösung zu erhalten, die Laktose als Präzipitat (9, 10) und eine oder mehrere Chargen Mutterlauge (15) und gegebenenfalls eine oder mehrere Chargen Waschwasser (16, 17) umfasst, um die ausgefällte kristallisierte Laktose (11, 12) zu waschen und zu trennen;
**dadurch gekennzeichnet, dass** die Anordnung (30) ferner Folgendes umfasst:
- eine Mutterlaugen-Recyclingleitung (31), die konfiguriert ist, um den Konzentrationsdurchgang (IV) mit einer ersten Vorratslösung des Erst- Zweit- und/oder Folge- Laktosekristallisationsdurchgangs durchzuführen, umfassend:
- einen ersten Sammeltank (18), der zum Sammeln der einen oder mehreren Chargen von Mutterlauge (15) und gegebenenfalls der einen oder mehreren Chargen von Waschwasser (16, 17) als erster Vorrat der Erstdurchgangs-Kristallisations-Laktoselösung konfiguriert ist;
- eine Entmineralisierungseinheit (34), die zum Entmineralisieren (20) des ersten Vorrats der Erstdurchgangs-Kristallisations-Laktoselösung konfiguriert ist, um eine entmineralisierte Laktoselösung des ersten Kristallisierungsdurchgangs zu erhalten;
- eine Konzentrationseinheit (36), die zum Konzentrieren (21) der entmineralisierten Erstdurchgangs-Kristallisations-Laktoselösung konfiguriert ist, um eine konzentrierte entmineralisierte Erstdurchgangs-Kristallisations-Laktoselösung zu erhalten,
- einen dritten Sammelbehälter, der zum Sammeln der konzentrierten demineralisierten Erstdurchgangs-Laktoselösung konfiguriert ist, wodurch ein zweiter Vorrat der demineralisierten Erst-, Zweit- und/oder Folge-Kristallisationslösung erhalten wird;
mindestens eine Pumpe (37) zum Pumpen der wässrigen Lösung, die Laktose umfasst, durch die Mutterlaugen-Recyclingleitung (31); und
eine Steuerung (38), die zum Durchführen eines Verfahrens nach einem der Ansprüche 1 bis 15 konfiguriert ist,
wobei der erste Sammeltank (18), die Entmineralisierungseinheit (34), die Konzentrationseinheit (36) und der dritte Sammeltank sequentiell auf einem Strömungspfad in der Mutterlaugen-Recyclingleitung (31) angeordnet sind, um einen ersten Vorrat der Erst-Zweit- oder Folgedurchgangs-Laktosekristallisationslösung zu ermöglichen, die Mutterlaugen-Recyclingleitung (31) in einem Batch-Prozess zu durchlaufen, wodurch ein zweiter Vorrat der konzentrierten entmineralisierten Erst- oder Folgedurchgangs-Laktosekristallisationslösung in einer Menge akkumuliert wird, die für die Verwendung in einem weiteren folgenden Laktosekristallisationsdurchgang (II) ausreichend ist.

13. Anordnung nach Anspruch 12, wobei die Mutterlaugen-Recyclingleitung (31) ferner eine Pasteurisierungseinheit (35) umfasst, die zum Pasteurisieren (19) des ersten Vorrats der Erstdurchgangs-Kristallisationslösung konfiguriert ist; wobei die Pasteurisierungseinheit (35) nach dem ersten Sammelbehälter (18) auf dem Strömungspfad angeordnet ist.

14. Anordnung nach einem der Ansprüche 12 oder 13, wobei die Mutterlaugen-Recyclingleitung (31) ferner einen zweiten Sammeltank (22) umfasst, der konfiguriert ist, um eine demineralisierte Erstdurchgangs-Kristallisationslösung aufzunehmen und zu akkumulieren, bevor die konzentrierte demineralisierte Erstdurchgangs-Kristallisationslösung auf die Laktosekristallisationsleitung (33) übertragen wird.

15. Anordnung nach einem der Ansprüche 12 bis 14, wobei die Steuerung (38) konfiguriert ist, um die Kristallisation von Laktose (8) bis mindestens 50 % der vorhandenen Gesamtmenge an Laktose zu erlauben, die in der wässrigen Lösung, die Laktose umfasst, kristallisiert wird.

## Revendications

1. Procédé d'obtention de lactose cristallisé (14), le procédé comprenant :
i) lors d'un premier passage de cristallisation (II) :
- la soumission d'une solution aqueuse comprenant du lactose à un procédé de cristallisation de lactose (9-12) afin d'obtenir du lactose cristallisé (14) et une liqueur mère (15), et,
- éventuellement, le lavage dudit lactose cristallisé (11, 12) produisant ainsi au moins un lot d'eau de lavage comprenant du lactose (16, 17),
- la récupération de ladite liqueur mère (15) et, lorsqu'il est présent, dudit au moins un lot de ladite eau de lavage (16, 17) pour obtenir ainsi une première réserve d'une solution de lactose de premier passage de cristallisation ;
ii) la réalisation dans un procédé discontinu (19-21) de ladite première solution de réserve d'un passage de concentration (IV) comprenant
- la déminéralisation (20) de ladite solution de lactose de premier passage de cristallisation, pour obtenir une solution déminéralisée de lactose de premier passage de cristallisation, et
iii) la concentration (21) de ladite solution déminéralisée de premier passage de lactose, pour obtenir ainsi une solution déminéralisée concentrée de lactose de premier passage de cristallisation ;
iv) la récupération de ladite solution déminéralisée concentrée de lactose de premier passage de cristallisation permettant ainsi d'obtenir une seconde solution de réserve, comprenant ladite solution déminéralisée concentrée de lactose de premier passage de concentration ; et
v) sur ladite seconde solution de réserve, on réalise un second passage de cristallisation (II), indépendamment dudit premier passage de cristallisation.

2. Procédé selon la revendication 1, comprenant dans ledit second passage de cristallisation (II)
- la soumission de ladite seconde solution de réserve audit procédé de cristallisation de lactose (9-12), afin d'obtenir un lactose cristallisé de second passage (14) et une liqueur mère de second passage (15),
- éventuellement, le lavage dudit lactose cristallisé de second passage (11, 12), permettant ainsi de produire au moins un lot d'eau de lavage comprenant du lactose (16, 17),
- éventuellement, la récupération de ladite liqueur mère du second passage de cristallisation (15) et, lorsqu'il est présent, dudit au moins un lot d'eau de lavage (16, 17), permettant ainsi d'obtenir une première réserve d'une solution de lactose de second passage de cristallisation.

3. Procédé selon l'une quelconque des revendications 1 ou 2, dans lequel ledit second passage de cristallisation (II) est répété au moins une fois en tant que passage de cristallisation ultérieur (II).

4. Procédé selon l'une quelconque des revendications 1 à 3, dans lequel ladite première solution de réserve dudit second passage de cristallisation (II) est en outre soumise à un procédé discontinu (19-21) audit passage de concentration (IV), au moins une fois.

5. Procédé selon l'une quelconque des revendications 1 à 4, dans lequel la cristallisation du lactose (8) est autorisée jusqu'à ce qu'au moins 50 % du lactose total présent dans ladite solution aqueuse comprenant du lactose ou n'importe laquelle de ladite réserve des solutions de lactose desdits premier ou second passages, voire d'un passage ultérieur, soit cristallisée.

6. Procédé selon l'une quelconque des revendications 1 à 5, dans lequel ladite première réserve de ladite solution déminéralisée de lactose des premier ou deuxième passages, voire d'un passage ultérieur, est recueillie dans un réservoir de récupération (22) avant de subir une autre concentration (21) et une autre cristallisation lors dudit second passage de cristallisation (voire lors d'un passage ultérieur (II)), afin d'obtenir du lactose cristallisé.

7. Procédé selon l'une quelconque des revendications 1 à 6, dans lequel au moins deux lots de la première réserve de ladite solution de lactose desdites solutions de lactose de premier passage ou de deuxième passage sont mélangés dans ledit réservoir de récupération (22) avant de subir, dans un procédé discontinu, lesdites concentration (21) et cristallisation dans ledit passage de cristallisation ultérieur (II), afin d'obtenir du lactose cristallisé.

8. Procédé selon l'une quelconque des revendications 1 à 7, dans lequel la solution aqueuse comprenant du lactose est du lactosérum ou un perméat de lactosérum (1).

9. Procédé selon l'une quelconque des revendications 1 à 8, dans lequel ladite déminéralisation (20) est réalisée par nanofiltration.

10. Procédé selon l'une quelconque des revendications 1 à 9, dans lequel le lactosérum ou le perméat de lactosérum est soumis à une étape de prétraitement (I) permettant ainsi d'obtenir une solution aqueuse contenant du lactose, ladite étape de prétraitement (I) comprenant :
a) la purification du lactosérum ou du perméat de lactosérum pour éliminer au moins un composé parmi une protéine, des polypeptides, des polysaccharides, des lipides ou de la riboflavine, et
b) la concentration dudit lactosérum ou dudit perméat de lactosérum.

11. Procédé selon les revendications 12 ou 13, dans lequel ladite purification du lactosérum ou du perméat de lactosérum (a) est réalisée par ultrafiltration.

12. Agencement (30) d'obtention de lactose cristallisé (14) à partir d'une solution aqueuse comprenant du lactose, ledit agencement comprenant :
- une conduite de cristallisation de lactose (33), conçue pour obtenir du lactose cristallisé à partir de la solution aqueuse comprenant du lactose sous forme de précipité (9, 10) et au moins un lot de liqueur mère (15) et, éventuellement, au moins un lot d'eau de lavage (16, 17) à partir du lavage et de la séparation dudit lactose cristallisé précipité (11, 12) ;
**caractérisé en ce que** ledit agencement (30) comprenant en outre :
- une conduite de recyclage de liqueur mère (31), conçue pour exécuter ledit passage de concentration (IV) sur une première solution de réserve des premier et deuxième passages et/ou d'un passage ultérieur de cristallisation de lactose, comprenant :
- un premier réservoir de récupération (18), conçu pour la récupération dudit au moins un lot de liqueur mère (15) et, éventuellement, dudit au moins un lot d'eau de lavage (16, 17) en tant que première réserve de ladite solution de lactose de premier passage de cristallisation ;
- une unité de déminéralisation (34), conçue pour la déminéralisation (20) de ladite première réserve de ladite solution de lactose de premier passage de cristallisation, pour obtenir une solution déminéralisée de lactose de premier passage de cristallisation ;
- une unité de la concentration (36), conçue pour la concentration (21) de ladite solution déminéralisée de lactose de premier passage de cristallisation, pour obtenir une solution déminéralisée concentrée de lactose de premier passage de cristallisation,
- un troisième réservoir de récupération, conçu pour la récupération de ladite solution déminéralisée concentrée de lactose de premier passage, ce qui permet d'obtenir une second réserve de ladite solution déminéralisée de lactose des premier et deuxième passages et/ou d'un passage de cristallisation ultérieur ;
au moins une pompe (37), destinée au pompage de ladite solution aqueuse comprenant du lactose à travers ladite conduite de recyclage de liqueur mère (31) ; et
un contrôleur (38), conçu pour exécuter un procédé selon l'une quelconque des revendications 1 à 15,
ledit premier réservoir de récupération (18), ladite unité de déminéralisation (34), ladite unité de concentration (36) et ledit troisième réservoir de récupération étant agencés de manière séquentielle sur une voie d'écoulement dans ladite conduite de recyclage de liqueur mère (31) pour permettre à une première réserve de ladite solution de lactose de premier passage ou de passage ultérieur de cristallisation de lactose de traverser ladite conduite de recyclage de liqueur mère (31) selon un procédé discontinu, permettant ainsi d'accumuler une second réserve de ladite solution déminéralisée concentrée de lactose de premier passage ou de passage ultérieur de cristallisation en quantité suffisante pour une utilisation lors d'un autre passage ultérieur (II) de cristallisation de lactose .

13. Agencement selon la revendication 12, dans lequel ladite conduite de recyclage de liqueur mère (31) comprend en outre une unité de pasteurisation (35), conçue pour la pasteurisation (19) de ladite première réserve de ladite solution de lactose de premier passage de cristallisation ; ladite unité de pasteurisation (35) étant située après ledit premier réservoir de récupération (18) sur ladite voie d'écoulement.

14. Agencement selon l'une quelconque des revendications 12 ou 13, dans lequel ladite conduite de recyclage liqueur mère (31) comprend en outre un deuxième réservoir de récupération (22), conçu pour la réception et pour l'accumulation de la solution déminéralisée de lactose de premier passage de cristallisation avant le transfert de ladite solution déminéralisée concentrée de lactose de premier passage de cristallisation vers ladite conduite de cristallisation de lactose (33).

15. Agencement selon l'une quelconque des revendications 12 à 14, dans lequel le contrôleur (38) est conçu pour permettre la cristallisation de lactose (8) jusqu'à ce qu'au moins 50 % du lactose total présent dans ladite solution aqueuse comprenant du lactose soit cristallisé.
